(19) Europäisches Patentamt
European Patent Office
Office européen des brevets

(11) Publication number: **0 385 491 A1**

# (12) EUROPEAN PATENT APPLICATION

(21) Application number: **90104069.1**

(51) Int. Cl.[5]: **C07D 295/088, C07B 57/00**

(22) Date of filing: **02.03.90**

(30) Priority: **03.03.89 IT 1963789**

(43) Date of publication of application:
**05.09.90 Bulletin 90/36**

(84) Designated Contracting States:
**BE DE DK ES FR GB GR IT LU NL**

(71) Applicant: **MAGIS FARMACEUTICI S.p.A.**
**Via Cacciamali 34/36/38**
**I-25125 Brescia(IT)**

(72) Inventor: **Puricelli, Laura**
**Residing in Via Taramelli, 7**
**I-25100 Brescia(IT)**

(74) Representative: **Gervasi, Gemma et al**
**NOTARBARTOLO & GERVASI Srl Viale**
**Bianca Maria 33**
**I-20122 Milan(IT)**

## (54) Optically active isomer of cloperastine possessing antitussive activity, a process for its preparation and pharmaceutical compositions which contain it.

(57) The present invention relates to the optical isomers of cloperastine, a compound of formula:

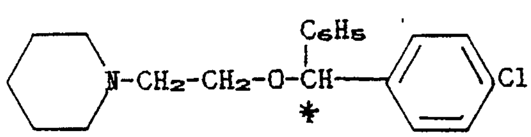

(I)

Because of the presence of the asymmetric carbon atom marked with the asterisk, the compound (I) exists in its dextrorotatory and levorotatory isomer forms.
The invention provides a process for the optical resolution of raceme DL-cloperastine and pharmaceutical compositions containing L-cloperastine.

EP 0 385 491 A1

# OPTICALLY ACTIVE ISOMER OF CLOPERASTINE POSSESSING ANTITUSSIVE ACTIVITY, A PROCESS FOR ITS PREPARATION AND PHARMACEUTICAL COMPOSITIONS WHICH CONTAIN IT

## Description of the invention

It is well known that both from the biochemical and pharmaceutical aspect the spatial structure of a molecule often determines its biological significance and its activity relative to the possibility of interaction with the biological receptor.

Organic chemistry defines optical isomers as those compounds the molecules of which lack planes or centres of symmetry. If the constituent atoms or atomic groups of the molecule are spatially arranged so as to define two molecular species which differ from each other in the same manner as an object differs from its specular image, these are known as optical antipodes or enantiomers.

These latter rotate the plane of polarized light through an amount which in the two cases is identical in absolute value but is of opposite sign (+ and -). The chemical properties of enantiomers are superposable except when they take part in reactions with other optically active molecules, however their biological properties are considerably influenced.

Optical isomerism influences the activity of the most widely differing drugs in terms of structure and activity, for many drugs it has beef found that the activity differences between two enantiomers are of an essentially quantitative order, ie such that they manifest the same biological activity but to different extents.

According to the present invention it bas now been surprisingly found that cloperastine, an antitussive drug used widely in therapy, has a considerable therapeutic advantage in its levorotatory form compared with its raceme form.

L-cloperastine is used in pharmaceutical compositions generally in the form of the salt of a pharmacologically acceptable acid such as the hydrochloride, hydrobromide, aceturate, besylate, tebutate, teoclate, amsonate, bunapsilate, dibudinate, cromesilate, lauryl sulphate, hybenzate, fendizoate, cromacate, embonate (previously pamoate), methylsulphate, napadisilate, oxoglurate, steaglate, triclofenate, citrate or acetate.

The compound according to the invention can be formulated in pharmaceutical compositions in mixture with a pharmacologically acceptable support or diluent.

The pharmaceutical preparations can be in solid form (such as capsules, tablets, sugar-coated pills of instantaneous or delayed action, single-dose sachets); in liquid form (ready or extemporaneous solutions or suspensions); or as suppositories or injectable solutions.

In treating the cough, the compound of the invention can be administered orally and topically in posological units containing for example from 50 mg to 400 mg of active principle twice a day, or by injection in posological units containing for example from 30 mg to 100 mg of active principle twice a day.

The compound according to the present invention can be obtained from raceme cloperastine by conventional optical resolution methods, or can be obtained synthetically from optically active p-chloro-α-phenylbenzenemethanol by the process according to the invention.

According to the process of the invention the levorotatory isomer of p-chloro-a-phenylbenzenemethanol is reacted with N-(2-chloroethyl) piperidine to obtain the levo cloperastine or the same levo p-chloro-α-phenylbenzenemethanol with ethylene chlorohydrin in the presence of sulphuric acid, p-chloro-α-phenylbenzenemethyl-β-chloroethylether is obtained which combines with piperidine to form levorotatory cloperastine.

p-chloro-a-phenylbenzenemethanol is obtained by reacting p-chloro-a-phenylbenzenemethanol with phthalic anhydride. The p-chloro-a-phenylbenzenemethanol hydrogen phthalate which forms is resolved with brucine or with (-)-1 phenylethylamine

The process takes place in accordance with the following scheme:

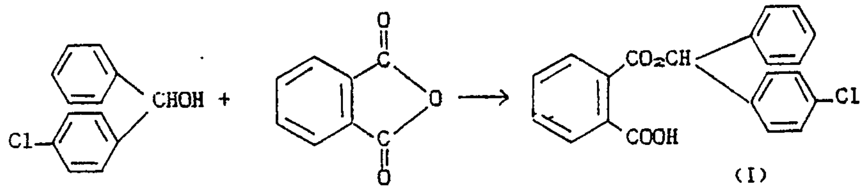

(I)

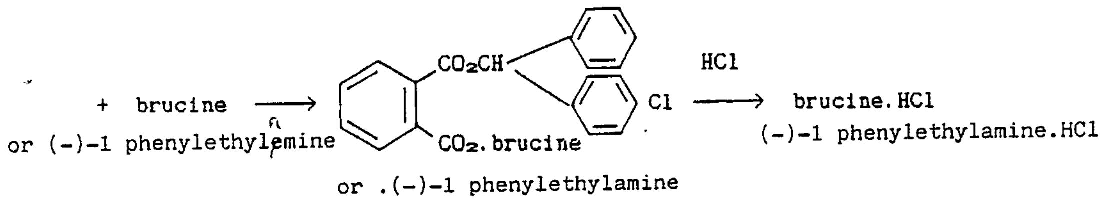


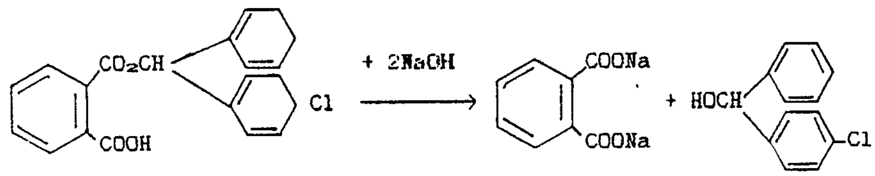


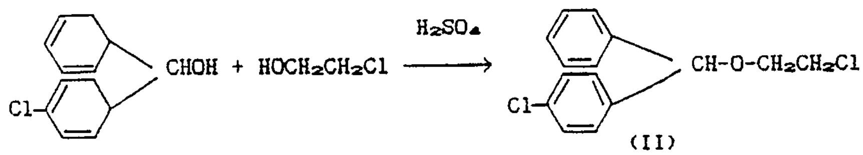


(II)

(II) +

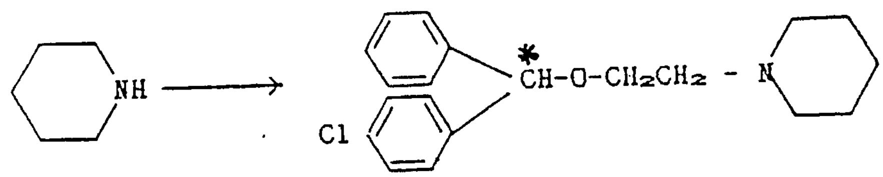

Or (II) +

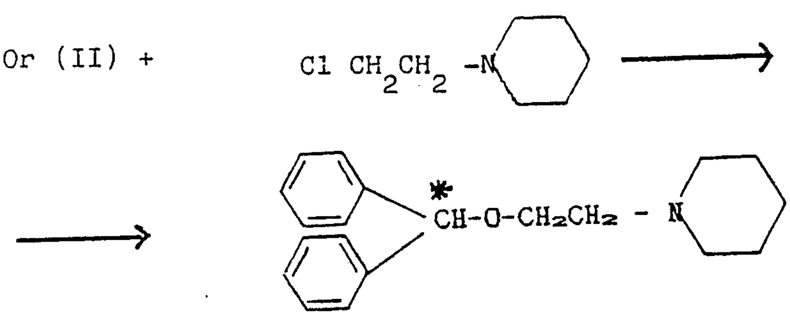

The following examples are given by way of non-limiting illustration of the invention.

EXAMPLE 1

DL-p-chloro-α-phenylbenzenemethanol

A) Preparation of p-chloro-α-phenylbenzenemethanol hydrogen phthalate:

A mixture of 218.68 g (1 mole) of p-chloro-α-phenylbenzenemethanol and 148 g (1 mole) of phthalic anhydride is heated for 12-15 hours in a flash placed in an oil bath heated to 110-115 °C.

The mixture is stirred mechanically during heating. On termination of the reaction the mixture is cooled and poured into a solution consisting of 8 litres of water containing 150 g (1.4 moles) of anhydrous sodium carbonate. The solid material slowly dissolves, and when the solution is clear a slight excess of dilute hydrochloric acid is added. p-chloro-a-phenylbenzenemethanol hydrogen phthalate precipitates and is filtered off and dried.

It is purified by crystallization from ether or petroleum ether (60-70 °C). The yield is quantitative.

B) Resolution:

394 g (1 mole) of brucine are added to a hot solution of 366.68 g (1 mole) of p-chloro-α-phenylbenzenemethanol hydrogen phthalate in 600 ml of acetone. The mixture is heated until a solution forms. It is cooled to ambient temperature. The brucine salt crystals (A) form.

The precipitate is filtered off, washed with 250 ml of acetone and then dried.

The filtrate and wash solution are pooled and reduced to about 50% of the original volume and then poured into dilute hydrochloric acid (in a slightly larger quantity than that calculated).

The crystals which form (B enantiomer) are filtered off, washed with cold water and dried. The A enantiomer is treated in the same manner with HCl to recover the phthalic ester. The two separated ester fractions have an angle of rotation in absolute alcohol of about ± 47°. The crystals (A) have an angle of rotation of about + 47°.

C) Hydrolysis:

The optically active D and L p-chloro-a-phenylbenzenemethanol are obtained by crystallization from ligroin after hydrolysing the phthalate with sodium hydroxide (2 moles of sodium hydroxide per mole of ester). The p-chloro-a-phenylbenzenemethanol separates because it is insoluble in water. It is dissolved in hot ligroin, dried with anhydrous sodium sulphate and then left to crystallize. Active compounds are obtained having approximate angles of rotation of between + 10° and + 9°, and between -10° and -9°.

EXAMPLE 2

L-p-chloro-a-phenylbenzenemethyl-β-chloroethylether

36 g (about 0.45 moles) of ethylene chlorohydrin, 5 ml of concentrated sulphuric acid and 35 ml of benzene are transferred into a 500 ml flask fitted with a stirrer, reflux condenser and dropping funnel.

The mixture is heated in a water bath after which a solution of 65.604 g (0.30 moles) of L-p-chloro-α-phenylbenzenemethanol in 65 ml of benzene is added over 30-50 minutes under efficient stirring.

The mixture is heated under reflux for about 4 hours while stirring. 35 ml of benzene are added to the mixture when cold. The benzene solution is washed with water and dried with anhydrous calcium chloride. It is filtered and the benzene evaporated. The residue is evaporated under vacuum at 174 °C at a reduced pressure of 1 mmHg.

About 70 g of product are obtained.

EXAMPLE 3

L-cloperastine

11.7 g of anhydrous piperidine (1.3 moles), 30 ml of isopropyl alcohol and 281.16 g (1 mole) of p-chloro-α-phenylbenzenemethyl-β-chloroethylether are added to a 500 ml flask fitted with a reflux condenser and dropping funnel. The mixture is heated to 80-90° C for 6 hours. It is cooled and poured into 300 ml of cold water.

The precipitate which forms is filtered off, dried and crystallized from absolute ethanol.

Elementary and spectral analyses confirm the structure of the compound, which has an angle of deviation of between -12° and -20°.

EXAMPLE 4

Resolution with tartaric acid

A) A mixture of 31.25 g (0.208 moles) of (+) tartaric acid and 450 ml of methanol are placed in a 1 litre flask and heated to boiling. 67.955 g (0.206 moles) of raceme cloperastine are carefully added to the hot solution.

The solution is allowed to cool and left standing for 24 hours. L (-) cloperastine (+) hydrogen tartrate salt separates. The product, of about 35 g, is filtered off and washed with a small volume of methanol.

The solution is reduced under vacuum to 175 ml. The resultant solution is left to crystallize for 24 hours. About 5 g of (-) cloperastine (+) hydrogen tartrate salt are obtained.

(+) cloperastine (+) hydrogen tartrate salt remains in the methanolic mother liquor.

The (-) cloperastine (+) hydrogen tartrate salt is dissolved in 400 ml of boiling methanol. When dissolved, the volume is reduced to about 300 ml, the solution cooled and left to crystallize for 24 hours. About 20 g of pure (-) cloperastine (+) hydrogen tartrate salt are obtained.

The pure (-) cloperastine (+) hydrogen tartrate salt is suspended in 90 ml of water and treated with 15 ml of 50% sodium hydroxide. The mixture is stirred energetically and extracted with chloroform. The chloroform extract is dried with anhydrous sodium sulphate and evaporated to dryness under vacuum.

In this manner about 15 g of pure L-cloperastine are obtained.

B) (+) cloperastine:

The methanolic mother liquor is evaporated to dryness under vacuum. The residual (+) cloperastine (+) hydrogen tartrate salt is suspended in 150 ml of water and treated with 25 ml of 50% sodium hydroxide solution.

The mixture is stirred energetically and extracted with chloroform. The chloroform layer is dried with anhydrous sodium sulphate and evaporated to dryness. (+) cloperastine is obtained in this manner.

The pharmacological properties of L-cloperastine compared with raceme cloperastine are illustrated briefly hereinafter.

EXAMPLE 5

A) Preparation of p-chloro phenylbenzenemethanol hydrogen phtalate.

A mixture of 218,68 g (1 mole) of p-chloro-α-phenyl benzenemethanol, 163 g (1.1 mole) of phtalic anhydride and 200 ml of pyridine is heated to 35° C for 3 hours.

The mixture is then mechanically stirred and then it is left standing for one night at room temperature.

At the end of the reaction the mixture is diluted with 2 l of ethylacetate and then poured into a solution consisting of 300 ml of 36% HCl and 1.5 l of water.

P-chloro-α-phenylbenzenemethanol hydrogen phtalate is then recovered from ethylacetate by solvent evaporation, and it is crystallized from toluene. The yield is quantitative.

B) Resolution

121,2 g of (-) 1 phenylethylamine are added to a hot solution of 2.5 l of absolute ethanol, containing 367.8 g (1 mole) of p-chloro-α-phenyl benzenemethanol hydrogen phtalate.
The mixture is then stirred and heated until a solution forms.
Then the mixture is cooled to room temperature: crystals of (-) 1-phenylethylamine salt form (A).
The precipitate is then filtered, washed with 600 ml of absolute ethanol and then dried.

C) HYDROLISIS

The filtrate and wash solution are pooled and 120 g of 50% sodium hydroxide are added to this solution.
The mixture is heated for 1 hour to 70° C and then it is cooled.
Sodium phtalate precipitates and then it is removed by means of filtration.
Ethanol is evaporated from the solution obtained; the residue is treated with benzene and washed with diluted HCl in a slightly larger quantity than that necessary to neutralize (-) 1-phenylethylamine.
The benzenic phase is then washed with water until neutral pH.
The enantiomer B is recovered from benzene evaporation and it is purified by crystallization with n-hexane.
The diasteroisomer A is then hydrolized with sodium hydroxide in order to recover enantiomer A in the same manner as enantiomer B.
Optical destro and levo isomers are obtained characterized by having rotation angles ranging respectively from +5.5 to +6° C and from -5.5 to -6° C.

EXAMPLE 6

(-) Cloperastine

65.604 g (0.30 mole) of (-) p-chloro-α-phenylbenzenemethanol and 175 ml of toluene are transferred into a 500 ml flask fitted with a reflux condenser, the solution is then heated to 100° C.
28 g (0.15 mole) of N-(2 chloroethyl) piperidine chlorohydrate and 256 g of 50% sodium hydroxide are then added to the reaction mixture.
After ten minutes 28 g (0.15 mole) of N-(2-chloroethyl) piperidine and 128 g of 50% sodium hydroxide are further added.
Every 10 minutes 14 g (0.075 mole) of N-(2-chloroethyl) piperidine are repeatedly added for four times.
At the end of the reaction the mixture is cooled and the toluene phase is washed with water.
The excess of N-(2 chloroethyl) piperidine is then removed by distillation at reduced pressure (70 °C at 15 mm Hg).
The residue consisting of (-) cloperastine is purified by distillation at 180° C with 0.15 mm Hg of pressure.
80 g of (-) cloperastine, exhibiting a rotation angle ranging from -6° to -6.5, are obtained.

Ammonia aerosol test:

Method: male albino guinea pigs of average weight 350 g were used divided into groups of 10 animals each.

Two groups of animals were treated with L-cloperastine hydrochloride orally at doses of 5 and 10 mg/kg respectively, two groups of animals were treated with raceme hydrochloride cloperastine at dose of 5 and 10 mg/kg respectively, and a last groups was used as the control.

30 minutes after administering each dose of the drug the animals were placed individually in a glass container into which ammonia vapour was fed.

The activity of the product under examination was evaluated by calculating the number of coughs emitted by the animals withjn a period of thirty minutes of observation. The table shows that levo-cloperastine has a very marked antitussive action which is superior to that of raceme cloperastine.

TABLE

| Antitussive activity study: ammonia aerosol test | | | | | | | |
|---|---|---|---|---|---|---|---|
| The table shows the average number of coughs ± SE in animals subjected to key treatments | | | | | | | |
| No. of animals | Treatment | Period of observation | | | | | |
| | | 5 min | 10 min | 15 min | 20 min | 25 min | 30 min |
| 10 | control | 23,72±1,13 | 27,55±2,97 | 24,79±5,26 | 19,97±1,39 | 27,33±2,73 | 22,20±4,61 |
| | levocloperastine | | | | | | |
| 10 | 5 mg/kg | 20,88±1,20 | 20,44±3,95 | 18,87±0,93 | 14,98±0,33 | 14,29±0,11 | 19,33±0,18 |
| 10 | 10 mg/kg | 18,04±1,37 | 16,23±3,13 | 1,81±4,27 | 1,06±2,22 | 6,59±3,11 | 6,44±3,30 |
| | raceme cloperastine | | | | | | |
| 10 | 5 mg/kg | 21,5 ±2,2 | 22,3±1,8 | 20,1±2,51 | 16,5±0,4 | 15,3±0,12 | 20,08±0,1 |
| 10 | 10 mg/kg | 19,04±13,4 | 17,5±4,1 | 8,5±3,4 | 6,3±3,1 | 8,1±3,15 | 10,5±3,5 |

## Claims

1. A process for preparing the D and L enantiomers of raceme cloperastine of formula:

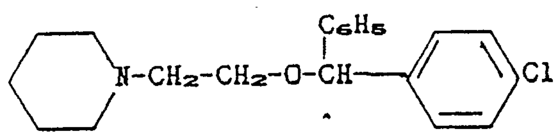

(I)

starting from raceme p-chloro-α-phenylbenzenemethanol, comprising the 4 following stages:

a) converting the raceme p-chloro-α-phenylbenzenemethanol by reaction with phthalic anhydride;

b) salifying the acid phtalate with brucine or (-) 1 phenylethylamine and then separating it into its two optical isomers by crystallization from acetone; recovering the acid phthalate isomers by acidifying the respective brucine or (-) 1 phenylethylamine salts with HCl;

c) hydrolysing the L or D acid phthalate with NaOH to obtain the L or D p-chloro-a-phenylbenzenemethanol respectively;

d) reacting the L or D p-chloro-α-phenylbezene methanol with N-(2 chloroethylpiperidine) to obtain directly destro or levo cloperastine or:

d′) reacting the L or D p-chloro-α-phenylbenzenemethanol with ethylene chlorohydrin in the presence of $H_2SO_4$ in benzene as reaction solvent, at the boiling temperature of the mixture to obtain compound III.

e) reacting compound (III) obtained in d′) with piperidine in an alcoholic solvent at a temperature of between 80 and 90 °C for some hours to obtain the L or D isomer of cloperastine respectively.

2. A process for preparing the D and L enantiomers of cloperastine consisting of subjecting raceme cloperastine to salification with optically active organic acids, separating the two optical isomers in the form of their salts, and then converting the salts into L or D cloperastine with NaOH.

3. A process as claimed in claim 2, wherein the optically active acid used is (+) tartaric acid.

4. Pharmaceutical compositions possessing antitussive activity, comprising a therapeutically effective quantity of L-cloperastine either as such or in the form of a water-soluble or water-insoluble salt thereof.

5. Pharmaceutical compositions as claimed in claim 4, in the form of tablets, capsules, single-dose sachets, syrup, elixirs, droplets, suppositories or injectable solutions.

Claims for the following Contracting States: ES, GR

1. A process for preparing the D and L enantiomers of raceme cloperastine of formula:

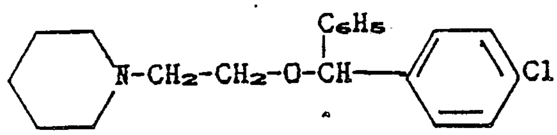

(I)

starting from raceme p-chloro-α-phenylbenzenemethanol, comprising the following stages:

a) converting the raceme p-chloro-α-phenylbenzenemethanol by reaction with phthalic anhydride;

b) salifying the acid phtalate with brucine or (-) 1 phenylethylamine and then separating it into its two optical isomers by crystallization from acetone; recovering the acid phthalate isomers by acidifying the respective brucine or (-) 1 phenylethylamine salts with HCl;

c) hydrolysing the L or D acid phthalate with NaOH to obtain the L or D p-chloro-a-phenylbenzenemethanol respectively;

d) reacting the L or D p-chloro-α-phenylbezene methanol with N-(2 chloroethylpiperidine) to obtain directly destro or levo cloperastine or:

d′) reacting the L or D p-chloro-α-phenylbenzenemethanol with ethylene chlorohydrin in the presence of $H_2SO_4$ in benzene as reaction solvent, at the boiling temperature of the mixture to obtain compound III.

e) reacting compound (III) obtained in d′) with piperidine in an alcoholic solvent at a temperature of between 80 and 90° C for some hours to obtain the L or D isomer of cloperastine respectively.

2. A process for preparing the D and L enantiomers of cloperastine consisting of subjecting raceme cloperastine to salification with optically active organic acids, separating the two optical isomers in the form of their salts, and then converting the salts into L or D cloperastine with NaOH.

3. A process as claimed in claim 2, wherein the optically active acid used is (+) tartaric acid.

European Patent Office

# EUROPEAN SEARCH REPORT

Application Number

EP 90 10 4069

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-1 153 436 (SOCIETE YAB) * Claims 1,3,5-10 * | 1,4,5 | C 07 D 295/088<br>C 07 B 57/00 |
| Y | GB-A- 670 622 (PARKE DAVIS & CO.) * Page 2, line 1 - page 3, line 47; examples; claims * | 1,4,5 | |
| A | GB-A- 797 473 (N.V. KONINKLIJKE PHARMACEUTISCHE FABRIEKEN) * Example 3; claims * | 2,3 | |
| A | GB-A- 905 993 (McNEIL LABORATIORIES INC.) * Examples; claims * | 2,3 | |

TECHNICAL FIELDS SEARCHED (Int. Cl.5)

C 07 D 295/00
C 07 B 57/00

The present search report has been drawn up for all claims

| Place of search | Date of completion of the search | Examiner |
|---|---|---|
| THE HAGUE | 06-06-1990 | HELPS I.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document